# EUROPEAN PATENT APPLICATION

(11) **EP 4 585 688 A1**
(43) Date of publication of application: **16.07.2025**
(21) Application number: 23881754.8
(22) Date of filing: 20.10.2023
(51) Int. Cl.: C12N 15/62, A61K 39/00, A61P 35/00, A61P 31/12

(54) **NUCLEIC ACID MOLECULE, FUSION PROTEIN AND MRNA VACCINE HAVING RECRUITMENT LIGAND FOR ENHANCING ANTIGEN-PRESENTING EFFECT**

(30) Priority: 24.10.2022 CN 202211302521
(71) Applicant: Westgene Biopharma Co., Ltd., Chengdu, Sichuan 610000 (CN)
(72) Inventor: SONG, Xiangrong, Chengdu, Sichuan 610000 (CN); HUANG, Hai, Chengdu, Sichuan 610000 (CN); WEI, Yuquan, Chengdu, Sichuan 610000 (CN); WEI, Xiawei, Chengdu, Sichuan 610000 (CN)
(74) Representative: karo IP
(86) International application number: PCT/CN2023/125695
(87) International publication number: WO 2024/088176

(57) **Abstract**

The present invention relates to the field of biomedicine, and mainly relates to a vaccine design method for enhancing an antigen-presenting effect. A target antigen and a ligand such as a polypeptide or a protein domain having an E3 ubiquitin ligase binding or recruitment function are jointly coded in a same nucleic acid sequence, thereby promoting the degradation of the antigen protein by means of a proteasome approach, increasing the number and abundance of antigen peptides having antigen epitopes, and forming more peptide-MHC (p-MHC) complexes, and the complexes are presented on the surfaces of the cells, thereby enhancing subsequent immune response, and exerting an efficient tumor immunotherapy effect. The nucleic acid, the protein and the polypeptide vaccine provided have an efficient antigen-presenting effect and strong immunogenicity, and have good clinical application prospects.

## Description

### Technical Field

This invention belongs to the field of biomedicine. It specifically relates to nucleic acid molecules, fusion proteins, and vaccines that can enhance antigen presentation, with a particular focus on mRNA vaccines.

### Background

mRNA vaccines are a novel type of vaccine known for their high versatility, efficacy, rapid construction, and ease of mass production. They hold significant clinical value and potential applications. The global spread of the COVID-19 pandemic has greatly accelerated the development of mRNA vaccines. Emerging nucleic acid vaccines (mRNA or DNA) have shown a distinct advantage in terms of timeliness as emergency vaccines. In fact, using mRNA vaccines to induce anti-tumor immune effects has shown great promise, with several research results entering clinical trials.

mRNA tumor vaccines introduce tumor antigens in the form of mRNA into patients through the translation mechanism of antigen-presenting cells (APCs). These antigens stimulate T cells in the patients, activating specific cellular immune responses for tumor treatment. Currently, no mRNA tumor vaccines are commercially available. However, 19 clinical trials have been registered globally, with the fastest reaching phase II clinical trials. Despite this progress, existing mRNA tumor vaccines need optimization in enhancing antigen presentation efficiency and T cell immune response. This includes improvements in mRNA sequence design and synthesis, translation efficiency and stability, antigen presentation efficiency, and T cell immune response strength. These optimizations aim to enhance anti-tumor immunotherapeutic activity and accelerate clinical translation.

mRNA tumor vaccines primarily rely on APCs, especially dentritic cells (DCs), to translate mRNA into target antigen proteins. Some of these antigen proteins undergo ubiquitination and degradation via the proteasome pathway, generating a large number of antigen peptides with antigen epitopes. These antigen peptides are transported to the endoplasmic reticulum (ER) by the transporter associated with antigen processing (TAP) and are modified by N-terminal aminopeptidases ERAP1 and ERAP2. They are then loaded onto MHC-I proteins, transported to the cell surface, and recognized by antigen-specific CD8+ T cells, presenting antigen epitope information to CD8+ T cells. Alternatively, secreted antigen proteins can be absorbed by professional antigen-presenting cells in tissues or lymph nodes. These proteins are degraded into numerous antigen peptides via the lysosomal degradation pathway. Subsequently, these peptides replace CLIP (MHC-II class molecule related invariant chain peptides) in the antigen-binding groove of MHC-II molecules, forming stable antigen peptide-MHC-II complexes. These complexes are then transported to the cell membrane for recognition by CD4+ T cells, presenting antigen epitope information to CD8+ T cells. Additionally, APCs can present internalized exogenous antigens on the cell surface through the MHC-I pathway, known as "cross-presentation". This cross-presentation is crucial for mRNA vaccines to induce cytotoxic T cell (CTL) and B cell responses.

To effectively induce adaptive immune responses, translated antigens in APCs (particularly DCs) need to be presented via MHC-I and MHC-II molecules. Therefore, the antigen presentation process is a critical factor influencing the immune efficacy of mRNA vaccines. All endogenous proteins are subject to MHC-I antigen presentation, but only a portion of intracellular antigens are presented by MHC-II molecules. However, the MHC-I antigen presentation process is typically inefficient, with only about one in ten thousand antigen peptides being presented, even for high-affinity MHC-I ligands. Thus, achieving efficient presentation of both MHC-I and MHC-II antigens is of great significance for the development of mRNA vaccines. Current strategies included co-delivering MHC molecules with antigens or targeting antigens to MHC molecules to enhance presentation efficiency. Studies reported that the combined use of DNA molecules encoding HPV-16 E7 and DNA vaccines encoding xenogeneic MHC molecules enhanced the cross-presentation mechanism. This significantly increased E7-specific immune responses and anti-tumor effects.

The series of steps in antigen processing and presentation affected the quantity of epitope/MHC complexes presents on the cell surface. Besides using MHC-I and MHC-II molecules to enhance antigen presentation, increasing the number of antigen peptides with epitopes through degradation is also an important and effective means to enhance antigen presentation. Most MHC-I antigen peptides are typically generated through a proteasome-dependent process. Strategies to increase proteasome-dependent degradation of antigen proteins improves MHC-I presentation of antigens. This enhancement leads to stronger E7-specific CD8+ T cell immune responses, significantly improving vaccine efficacy. Therefore, targeting the proteasome to increase antigen protein degradation might provide a novel approach for the development of nucleic acid-based drugs.

### Description of the Invention

To address the issues present in existing technology, this invention provides a vaccine design strategy to enhance antigen presentation. It is applied to the structural sequence design and preparation of nucleic acid, protein, and peptide vaccines. This offeres a new approach to improve the immunogenicity of vaccines and provided new ideas for the development of novel therapeutic methods.

This invention is based on the following work by the inventors:
They used OVA as a model antigen protein and selected three E3 ligase peptide ligands, MDM2, VHL, and Keap1, to couple with it. These ligands included PMI, VHL Ligand (referred to as VHLL), and Keap1 binding element (referred to as Keap1B). They also selected the helical residues 17-26 of P53 as the E3 ligase ligand (referred to as P53B) based on the structure of the MDM2-P53 complex. Further validation showed that E3 ubiquitin ligase binding or recruiting ligands enhanced the proteasome-dependent degradation of OVA antigen protein inside cells. This improved the tumor inhibitory effect and anti-tumor activity of mRNA tumor vaccines. The technical solution of this invention could be applied to the design of other mRNA tumor vaccines. Studies in various tumor models confirmed its effectiveness in promoting antigen presentation and activating specific T cells to kill tumor cells, achieving efficient tumor immunotherapy. This technical solution could also be applied to the design of preventive mRNA vaccines to achieve enhanced immunogenicity.

One aspect of this invention provided a nucleic acid molecule.

The open reading frame of the nucleic acid molecule contained at least one antigen element and at least one E3 ligand element. The E3 ligand element is a binding or recruiting ligand of E3 ubiquitin ligase.

The E3 ubiquitin ligase in the nucleic acid molecule is optionally selected from one or more of VHL (Von Hippel-Lindau), MDM2, CRBN, IAPs, RNF, β-TrCP, DCAF, Keap1, or their truncated or extended forms.

Furthermore, the E3 ubiquitin ligase is selected from RING type, HECT type, and RBR type.

Furthermore, the preferred E3 ubiquitin ligase in the nucleic acid molecule is one or more of VHL, MDM2, β-TrCP, Keap1, or their truncated or extended forms.

Furthermore, the binding or recruiting ligand of the E3 ubiquitin ligase bound to one or more E3 ubiquitin ligases.

Furthermore, the amino acid sequence corresponding to the binding or recruiting ligand of Keap1 is LDPETGEYL or a sequence with more than 60% homology.

Furthermore, the amino acid sequence corresponding to the binding or recruiting ligand of β-TrCP is DRHDSGLDSM or a sequence with more than 60% homology.

Furthermore, the amino acid sequence corresponding to the binding or recruiting ligand of VHL is at least one of LAP(OH)YI or ALAPYIP or a sequence with more than 60% homology.

Furthermore, the amino acid sequence corresponding to the binding or recruiting ligand of MDM2 is one or more of ETFSDLWKLL, TSFAEYWNLLSP, LTFEHYWAQLTS, TNWYANLEKLLR, TAWYANFEKLLR, DWWPLAFEALLR, CNCKAPETALCARRCQQH, or CNCKAPETFLCYWRCLQH or a sequence with more than 60% homology.

Furthermore, in the nucleic acid molecule, the antigen element and the E3 ligand element were connected in any form of E3 ligand element-antigen element, antigen element-E3 ligand element, E3 ligand element-antigen element-E3 ligand element, or antigen element-E3 ligand element-antigen element.

Furthermore, the antigen element and the E3 ligand element were connected by a linker. The amino acid sequence of the linker is one or more of GGGGS, (GGGGS)3, (GGGGS)6, (GGS)10, or (GSG)10.

Furthermore, the antigen element included natural antigen proteins, antigen polypeptides, or their truncated forms.

Furthermore, it included at least one antigen epitope.

Furthermore, the antigen element could include 1 to 20 antigen epitopes.

Furthermore, the antigen epitope is a T cell antigen determinant or T cell antigen epitope.

These epitopes originated from tumor antigen peptides, autoimmune disease antigen peptides, or pathogenic microorganism antigen peptides.

Tumor antigens or antigen peptides could be antigens from gene mutations, tissue-specific differentiation antigens, overexpressed antigens, cancer-testis antigens, common antigens, or oncogenic viral antigens.

Furthermore, gene mutation-induced antigens included one or more of p53, ras, β-catenin, CDK4, CDC27, or α-actinin-4.

Furthermore, tissue-specific differentiation antigens included one or more of tyrosinase, TRP1/gp75, TRP2, gp100, Melan-A/MART1, gangliosides, or PSMA.

Furthermore, overexpressed antigens included one or more of HER2, WT1, EphA3, EGFR, or CD20.

Furthermore, cancer-testis antigens included one or more of MAGE, BAGE, GAGE, or NY-ESO-1.

Furthermore, common antigens included one or more of Telomerase or Survivin.

Furthermore, oncogenic viral antigens included one or more of EBV, HPV, HBV, human herpesvirus, or Merkel cell polyomavirus.

The nucleic acid molecule further included a signal peptide coding region.

It also included a promoter, a 5' untranslated region (UTR), a 3' UTR, and a polyA tail.

The promoter is T7 or SP6.

Furthermore, the nucleic acid molecule could be DNA, ASO, siRNA, miRNA, mRNA, or aptamer.

Furthermore, the nucleic acid molecule, nucleic acid is mRNA.

The invention provided a nucleic acid vaccine containing the above nucleic acid molecule and optionally pharmaceutically acceptable excipients or auxiliary components.

Furthermore, the nucleic acid vaccine is an mRNA vaccine, with auxiliary components being nanocarriers for delivering the mRNA.

Furthermore, excipients included one or more of injection buffer media, lyophilization, or freeze-drying protectants.

Furthermore, nanocarriers included one or more of liposomes, nanoparticles, microspheres, or lipid nanocarriers. Nanocarriers were prepared using at least one lipid material such as DOTAP, DOTMA, DOTIM, DDA, DC-Chol, CCS, diC14-amidine, DOTPA, DOSPA, DTAB, TTAB, CTAB, DORI, DORIE and derivatives, DPRIE, DSRIE, DMRIE, DOGS, DOSC, LPLL, DODMA, DDAB, Dlin-MC3-DMA, CKK-E12, C12-200, DSPC, DMG-PEG, DOPE, phosphatidylethanolamine, phosphatidylcholine, and cholesterol.

Furthermore, the mass ratio of lipid material to mRNA in the mRNA vaccine is (0.5 to 50): 1, preferably (2 to 10):1.

Furthermore, the mRNA vaccine is formed by self-assembly using microfluidic devices.

Or by incubating the nanocarrier with mRNA.

Additionally, the invention also provided a method for preparing the nucleic acid vaccine and a protein encoded by any of the nucleic acid molecules mentioned above.

Additionally, a protein or polypeptide vaccine is provided, where the protein acted as the antigen component.

Additionally, this vaccine also included pharmaceutically acceptable excipients or auxiliary components, as defined in the nucleic acid molecules mentioned above.

It further included immunoadjuvants, which could be one or more of incomplete Freund's adjuvant, complete Freund's adjuvant, aluminum hydroxide adjuvant, aluminum phosphate adjuvant, emulsified adjuvant, liposome adjuvant, or microbial adjuvant.

The invention also provided a vector carrying the nucleic acid molecule mentioned above.

Additionally, the vector could be a eukaryotic or prokaryotic vector.

Additionally, it included one or more selected from plasmid vectors, adenoviral vectors, lentiviral vectors, and adeno-associated viral vectors.

Additionally, the invention provided a vector vaccine that included an active ingredient. The active ingredient is obtained by loading the nucleic acid molecule into the vector.

Additionally, the invention provided a pharmaceutical composition that included the nucleic acid molecule, the nucleic acid vaccine, the mRNA vaccine prepared by the method mentioned above, the protein, the protein or polypeptide vaccine, or the vector vaccine, along with pharmaceutically acceptable excipients.

Additionally, the invention also provided the use of the nucleic acid molecule, nucleic acid vaccine, mRNA vaccine prepared by the mentioned method, protein, protein or polypeptide vaccine, vector vaccine, and pharmaceutical composition in preparing medicines for preventing or treating related diseases.

Furthermore, the disease is preferably a tumor.

The beneficial effects of the present invention are as followed.

This invention provided a vaccine design method to enhance antigen presentation, applicable to the sequence design and preparation of nucleic acid, protein, and polypeptide vaccines.

By co-encoding the target antigen with polypeptides or protein domains having E3 ubiquitin ligase binding and recruitment functions in the same nucleic acid sequence, the invention ensured fusion expression of the antigen protein and the E3 ubiquitin ligase ligand after the nucleic acid molecule entered the cell. This promoted the degradation of the antigen protein through the proteasome pathway, increasing the quantity and abundance of antigen peptides with epitopes. These formed more peptide-MHC (p-MHC) complexes, which were then presented on the cell surface, enhancing subsequent immune responses and exhibiting efficient tumor immunotherapy effects.

The proteins or polypeptides, or the proteins or polypeptides encoded by the nucleic acid molecules of the invention, relied on the ubiquitin-proteasome pathway for antigen presentation in cells. By selecting and constructing different E3 ligase ligand sequences for modification, the invention screened E3 ubiquitin ligase ligands to efficiently recruit E3 ligase. This achieved ubiquitination and protein degradation of the proteins or polypeptides, thereby improving the antigen presentation of vaccine immunogens. Thus, the proteins, polypeptides, or nucleic acid vaccines provided by the invention demonstrated efficient antigen presentation and strong immunogenicity, with promising clinical application prospects.

### Figures Description

Figure 1. Particle size and PDI results of mRNA-LNP in Example 3.
Figure 2. Zeta potential results of mRNA-LNP in Example 3.
Figure 3. TEM image of OVA-P53B mRNA lipid nanoparticles in Example 3.
Figure 4. Activation of lymph node T cells by mRNA lipid nanoparticles in Example 4; Figure 4A shows early activation T cell data, and Figure 4B shows late activation T cell data.
Figure 5. Activation of spleen T cells by mRNA lipid nanoparticles in Example 4; Figure 5A shows early activation T cell data, and Figure 5B shows late activation T cell data.
Figure 6. Schematic of the immunization protocol in Example 4.
Figure 7. Tumor growth curves in E.G7-OVA tumor-bearing mice.
Figure 8. Representative photos of tumors in each treatment group.
Figure 9. Body weight-time curves of E.G7-OVA tumor-bearing mice.
Figure 10. Antigen presentation results of various mRNA lipid nanoparticles in mice in Example 5.
Figure 11. Specific CTL detection results mediated by various mRNAlipid nanoparticles in mice in Example 5. Figure 11A. Specific CTL detection results mediated by mRNA lipid nanoparticles in mouse lymph nodes. Figure 11B. Specific CTL detection results mediated by mRNA lipid nanoparticles in mouse spleen. Figure 11C. Specific CTL detection results mediated by mRNA lipid nanoparticles in mouse tumor tissues.
Figure 12. CTL generation results mediated by various mRNA lipid nanoparticles in mice in Example 6.
Figure 13. Detection data of ALT, AST, TP, CRE, LDH, and UREA in mouse serum in Example 7.

### Detailed Implementation

The following describes the embodiments of the invention in detail. The described embodiments are exemplary and are intended to illustrate the invention without limiting its scope.

The term "comprising" or "including" is open-ended and indicates the inclusion of stated elements without excluding other elements.

The term "optionally" means that the described event or condition may or may not occur, and includes instances where the event or condition does occur and where it does not.

The term "(XXXX)n" represents n number of connected XXXX units, where X represents amino acids. For example, "(GGGGS)3" represents GGGGSGGGGSGGGGS.

The term "fragment" refers to a target protein or polypeptide with N-terminal (N-terminus) or C-terminal (C-terminus) truncations, and/or internal deletions.

The terms "identity," "homology," or "similarity" describe the percentage of identical amino acids or nucleotides between two sequences compared to a reference sequence, determined using conventional methods. For example, see Ausubel et al., eds. (1995), Current Protocols in Molecular Biology, Chapter 19 (Greene Publishing and Wiley-Interscience, New York); and the ALIGN program (Dayhoff, 1978, Atlas of Protein Sequence and Structure 5: Suppl. 3, National Biomedical Research Foundation, ishington, D.C.). There are many algorithms for sequence alignment and determining sequence identity, including the Needleman-Wunsch algorithm (1970, J. Mol. Biol. 48: 443); the Smith-Waterman algorithm (1981, Adv. Appl. Math. 2: 482); the similarity search method of Pearson and Lipman (1988, Proc. Natl. Acad. Sci. 85: 2444); the Smith-Waterman algorithm (Meth. Mol. Biol. 70: 173-187, 1997); and the BLASTP, BLASTN, and BLASTX algorithms (Altschul et al., 1990, J. Mol. Biol. 215: 403-410). Computer programs utilizing these algorithms are also available, including but not limited to the ALIGN or Megalign (DNASTAR) software, WU-BLAST-2 (Altschul et al., Meth. Enzym., 266: 460-480, 1996), GAP, BESTFIT, BLAST (Altschul et al., above), FASTA, and TFASTA within the Genetics Computing Group (GCG) package, version 8, Madison, Wisconsin, USA; and the CLUSTAL program in the PC/Gene package from Intelligenetics, Mountain View, California.

In this document, the term "vector" typically refers to a nucleic acid molecule capable of self-replication within a suitable host. This vector transfers the inserted nucleic acid molecule into and/or between host cells. Vectors may include those primarily used for inserting DNA or RNA into cells, replicating DNA or RNA, and expressing DNA or RNA through transcription and/or translation. The vector can also encompass vectors with multiple of these functions. When introduced into a suitable host cell, the vector can transcribe and translate into polypeptides. Typically, the desired expression product is produced by culturing appropriate host cells containing the vector.

The term "pharmaceutical composition" generally refers to a unit dosage form that can be prepared by any method well-known in the pharmaceutical field. All methods involve combining the active ingredient with one or more auxiliary components. Compositions are usually prepared by thoroughly and uniformly mixing the active compound with liquid carriers, finely divided solid carriers, or both.

The term "pharmaceutically acceptable excipient" includes any solvent, solid excipient, diluent, or other liquid excipient suitable for the specific dosage form. These excipients are considered within the scope of the invention unless they cause adverse biological effects or interact harmfully with any other components of the pharmaceutical composition.

The term "administration" refers to introducing a predetermined amount of a substance to a patient through a suitable method. The nucleic acid molecules, nucleic acid vaccines, vectors, vector vaccines, polypeptides, antibodies or antigen-binding fragments, recombinant proteins, bispecific antibodies, protein or polypeptide vaccines, conjugates, or pharmaceutical compositions of the invention can be administered through any common route, provided they reach the intended tissue. Expected methods of administration include intraperitoneal, intravenous, intramuscular, and subcutaneous injections, though the invention is not limited to these methods. Preferably, the compositions of the invention are administered by intravenous or subcutaneous injection.

The term "E3 ubiquitin ligase" refers to enzymes that regulate various cellular processes by modulating the ubiquitination of regulatory proteins. All E3s have the capability to link target proteins and specific E2 enzymes. Known E3 ubiquitin ligases fall into two major categories: the HECT domain family and the RING domain family, as well as the U-box protein family. The HECT domain operates by forming a thioester bond with ubiquitin necessary for catalytic activity, while the RING domain provides a binding site for E2 and the substrate, facilitating the transfer of ubiquitin from E2 to the substrate.

The terms "binding or recruiting ligand of E3 ubiquitin ligase," "E3 ligand," "E3 ligand element," and "E3 ubiquitin ligase ligand" refer to natural or artificial polypeptides, proteins, or their truncated or extended forms, or compounds that can bind or recruit E3 ubiquitin ligase.

Since most peptides presented by MHC-I molecules originate from the degradation of antigen proteins, strategies to enhance the proteasomal degradation of antigen proteins have been shown to improve MHC-I presentation of antigens. This invention innovatively connects the recruiting ligand of E3 ubiquitin ligase to the antigen element, forming a fusion protein. This fusion protein can self-ubiquitinate through the ubiquitin-proteasome system (UPS) in vivo, initiating its ubiquitin-mediated degradation. This increases the quantity of ubiquitinated and degraded antigen proteins, enhancing the abundance of usable antigens. Consequently, it improves the direct presentation of target proteins or target epitopes by MHC-I, further enhancing the immune response induced by the vaccine.

The sequence design of mRNA drugs is crucial to their ultimate efficacy. Although the coding region of mRNA does not have as much plasticity as the non-coding region, the inherent adaptability of mRNA molecules allows for slight functional modifications in the coding region during design and synthesis to enhance efficacy. A critical step in the immune process involving mRNA vaccines is the antigen presentation of degraded peptides. Most peptides presented by MHC-I molecules originate from the degradation of antigen proteins. The ubiquitin-proteasome degradation mechanism achieves targeted degradation of target proteins. Based on this, the invention specifically increases the proteasome pathway degradation of target antigen proteins, enriching the pool of MHC-I presented peptides. The invention involves encoding polypeptide E3 ligase ligands into the mRNA molecule. By recruiting E3 ligases, the strategy induces rapid and efficient ubiquitination of the target antigen protein. This promotes the degradation of the target antigen protein through the proteasome pathway, increasing the abundance of pMHC complexes. This enhances the antigen presentation effect of mRNA drugs, triggering an efficient immune response and achieving potent tumor immunotherapy.

The invention designs and validates the following technical solution structures for vaccine preparation:
Nucleic Acid Molecule: The open reading frame (ORF) includes at least one antigen element and at least one E3 ligand element, where the E3 ligand element is a binding or recruiting ligand of E3 ubiquitin ligase.

Antigen element - (linker) - E3 ubiquitin ligase recruiting ligand element.

The recruiting ligand element of the E3 ubiquitin ligase is not directly or via linker elements connected to a ligand that binds the target protein in cells but is connected to the antigen element. Experiments have shown that this structure effectively increases the degradation quantity of antigen proteins, improving the abundance of usable antigens. This enhances the direct presentation of target proteins by MHC-I and further enhances the immune response induced by the vaccine.

When linker elements are present, the structures are: E3 ligand - linker - Antigen element, Antigen element - linker - Antigen element - linker - Antigen element - linker - E3 ligand, E3 ligand - linker - Antigen element - linker - E3 ligand, Antigen element - linker - E3, ligand - linker - Antigen element

The linker element includes one or more sequences from GGGGS, (GGGGS)₃, (GGGGS) ₆, (GGS)₁₀ 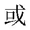 (GSG)₁₀. The amino acid sequences are one or more of GGGGS, (GGGGS)₃, (GGGGS) ₆, (GGS)₁₀ 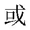 (GSG)₁₀.
GGGGS: GGGGS (Seq ID No.1) ;
(GGGGS)3: GGGGSGGGGSGGGGS (Seq ID No.2) ;
(GGGGS)6: GGGGSGGGGSGGGGSGGGGSGGGGSGGGGS (Seq ID No.3) ;
(GGS)10: GGSGGSGGSGGSGGSGGSGGSGGSGGSGGS (Seq ID No.4) ;
(GSG)10: GSGGSGGSGGSGGSGGSGGSGGSGGSGGSG (Seq ID No.5) _{∘}

The vaccine design strategy for enhancing antigen presentation can be applied to the structural sequence design and preparation of nucleic acid, protein, and polypeptide vaccines. The recruiting ligand element of the E3 ubiquitin ligase refers to any polypeptide, protein, or their nucleic acid encoding sequence that can bind to the E3 ubiquitin ligase.

The E3 ubiquitin ligase binding or recruiting ligand can bind with one or more E3 ubiquitin ligases. Common E3 ubiquitin ligase molecules include VHL, MDM2, CRBN, and IAPs. Common ligands for these E3 ubiquitin ligases include: VHL ligand (VHLL), amino acid sequence: ALAPYIP, Keap1 binding element, amino acid sequence: LDPETGEYI.

**Table 1: E3 Ligases and Their Peptide Ligands**

| E3 Ligase | Peptide ligands of E3 ligase | | |
|---|---|---|---|
| | Abbreviation | Property | Amino acid sequence |
| MDM2 | P53B | Natural | ETFSDLWKLL (Seq ID No.6) (The 17-26 positions of the alpha helix residue of P53) |
| | PMI | Manual | TSFAEYWNLLSP (Seq ID No.7) |
| | PDI | Manual | LTFEHYWAQLTS (Seq ID No.8) |
| | ^{D}PMI-*α* | Manual | TNWYANLEKLLR (Seq ID No.9) |
| | ^{D}PMI-*β* | Manual | TAWYANFEKLLR (Seq ID No.10) |
| | ^{D}PMI-*γ* | Manual | DWWPLAFEALLR (Seq ID No.11) |
| | Apamin | Manual | CNCKAPETALCARRCQQH ( Seq ID No.12) |
| | stingin | Manual | CNCKAPETFLCYWRCLQH ( Seq ID No.13) |
| VHL(Von Hippel-Lindau) | VHLL (Von Hippel-Lindau Ligand) | Natural | ALAPYIP (Seq ID No.14) |
| | | Manual | LAP(OH)YI (Seq ID No.15) |
| Keap1 | Keap1 B | Manual | LDPETGEYL (Seq ID No.16) |
| β-TrCP | β-TrCPB | Natural | DRHDSGLDSM (Seq ID No.17) |

The amino acid sequences for Keap1, β-TrCP, VHL, and MDM2 in Table 1 can use sequences with more than 60% homology.

The amino acid sequences for Keap1, β-TrCP, VHL, and MDM2 in Table 1 can use sequences with more than 70% homology.

The amino acid sequences for Keap1, β-TrCP, VHL, and MDM2 in Table 1 can use sequences with more than 80% homology.

The amino acid sequences for Keap1, β-TrCP, VHL, and MDM2 in Table 1 can use sequences with more than 90% homology.

The amino acid sequences for Keap1, β-TrCP, VHL, and MDM2 in Table 1 can use sequences with more than 95% homology.

The open reading frame (ORF) can also include a signal peptide coding sequence at the N-terminus. Typically, a Class I MHC transport signal sequence, the antigen source protein's own signal peptide, or other commonly used signal peptides in the field are used.

In practice, the antigen element contains at least one known or unknown antigen epitope. These epitopes can originate from tumors, autoimmune diseases, or infectious diseases. The antigen element includes polypeptides or proteins with at least one antigen epitope.

Among above, the antigen protein can be used directly as the antigen element. The number of antigen proteins can be one.

Among above, one or more antigen epitope peptides can be recombined to form a polypeptide molecule used as the antigen element.

Among above, these epitopes can originate from tumors, autoimmune diseases, or infectious diseases.

Among above, the antigen or antigen epitope peptides can derive from tumor antigens, viral pathogen antigens, or any combination.

Tumor antigens can be any tumor-specific antigens, tumor-associated antigens, or tumor neoantigens.

Examples of tumors include but are not limited to breast cancer, ovarian cancer, testicular cancer, pancreatic cancer, liver cancer, colorectal cancer, thyroid cancer, lung cancer, prostate cancer, kidney cancer, melanoma, squamous cell carcinoma, gastrointestinal adenocarcinoma, chronic myeloid leukemia, acute lymphoblastic leukemia, acute myeloid leukemia, chronic lymphocytic leukemia, promyelocytic leukemia, multiple myeloma, B-cell lymphoma, bladder cancer, head and neck cancer, esophageal cancer, brain cancer, pharyngeal cancer, tongue cancer, synovial sarcoma, neuroblastoma, uterine cancer, fibrosarcoma, myxosarcoma, liposarcoma, chondrosarcoma, osteosarcoma, chordoma, hemangiosarcoma, endothelial sarcoma, lymphangiosarcoma, synovioma, mesothelioma, Ewing's sarcoma, leiomyosarcoma, rhabdomyosarcoma, basal cell carcinoma, epidermoid carcinoma, adenocarcinoma, sweat gland carcinoma, sebaceous gland carcinoma, papillary carcinoma, papillary adenocarcinoma, cystadenocarcinoma, medullary carcinoma, bronchial carcinoma, renal cell carcinoma, liver tumor, cholangiocarcinoma, choriocarcinoma, seminoma, embryonal carcinoma, nephroblastoma, uterine cancer, cervical cancer, small cell lung cancer, epithelial cancer, glioma, astrocytoma, neuroectodermal tumor, craniopharyngioma, ependymoma, pineal tumor, hemangioblastoma, acoustic neuroma, oligodendroglioma, meningioma, brain or spinal cord tumor, neuroglioma, or retinoblastoma.

The one or more antigen epitopes can be one, two, or more epitopes from tumor-associated antigens. These tumor-associated antigens are selected from the following.

Kallikrein 4, Papillomavirus Binding Factor (PBF), Preferentially Expressed Antigen in Melanoma (PRAME), Wilm's Tumor-1 (WT1), Hydroxysteroid Dehydrogenase-Like 1 (HSDL1), Mesothelin, Cancer-Testis Antigen (NY-ESO-1), Carcinoembryonic Antigen (CEA), p53, Human Epidermal Growth Factor Receptor 2/Neuroreceptor Tyrosine Kinase (Her2/Neu), EpCAM (Epithelial Cell Adhesion Molecule), CA125 (Ovarian and Uterine Cancer Antigen), Folate Receptor α, Sperm Protein 17, Tumor-Associated Differentially Expressed Gene-12 (TADG-12), Mucin-16 (MUC-16), L1CAM (L1 Cell Adhesion Molecule), Mannan-MUC-1, Human Endogenous Retrovirus K (HERV-K-MEL), Kita-Kyushu Lung Cancer Antigen-1 (KK-LC-1), Human Cancer/Testis Antigen (KM-HN-1), Cancer-Testis Antigen (LAGE-1), Melanoma Antigen-A1 (MAGE-A1), Sperm Surface Zona Pellucida Binding Protein (Sp17), Synovial Sarcoma, X Breakpoint 4 (SSX-4), TAG-1 (Transient Axonal Glycoprotein-1), TAG-2, ENAH (Enabled Homolog), Mammaglobin-A, NY-BR-1, Breast Cancer Antigen, BAGE-1, B-Melanoma Antigen, MAGE-A1, MAGE-A2, Mucin-K, Synovial Sarcoma, X Breakpoint 2 (SSX-2), Paclitaxel Resistance-Related Gene-3 (TRAG-3), Avian Leukosis Virus Oncogene (c-myc), Cyclin B1, Mucin-1 (MUC1), p62, Survivin, CD45 (Leukocyte Common Antigen), Dickkopf WNT Signaling Pathway Inhibitor 1 (DKK1), Telomerase, Kirsten Rat Sarcoma Viral Oncogene Homolog (K-ras), G250, Intestinal Carboxylesterase, Alpha-Fetoprotein, Macrophage Colony-Stimulating Factor (M-CSF), Prostate-Specific Membrane Antigen (PSMA), Caspase-5 (CASP-5), Cytochrome C Oxidase Assembly Factor 1 Homolog (COA-1), O-Linked β-N-Acetylglucosamine Transferase (OGT), Osteosarcoma Amplified 9, Endoplasmic Reticulum Lectin (OS-9), Transforming Growth Factor Beta Receptor 2 (TGF-βRII), Murine Leukemia Glycoprotein 70 (gp70), Calcitonin-Related Polypeptide α (CALCA), CD274 (Programmed Death-Ligand 1), Mouse Double Minute 2 Homolog (mdm-2), Actin Alpha-4, Elongation Factor 2, Malic Enzyme 1 (ME1), Nuclear Transcription Factor Y Subunit C (NFYC), G Antigen 1, 3 (GAGE-1, 3), MAGE-A6, Cancer/Testis Antigen XAGE-1b, Six-Transmembrane Epithelial Antigen of Prostate 1 (STEAP1), PAP, Prostate-Specific Antigen (PSA), Fibroblast Growth Factor 5 (FGF5), Heat Shock Protein hsp70-2, MAGE-A9, Arginine-Specific ADP-Ribosyltransferase Family Member C (ARTC1), B-Raf Proto-Oncogene (B-RAF), Serine/Threonine Kinase, β-Catenin, Cell Division Cycle 27 Homolog (Cdc27), Cyclin-Dependent Kinase 4 (CDK4), Cyclin-Dependent Kinase 12 (CDK12), Cyclin-Dependent Kinase Inhibitor 2A (CDKN2A), Casein Kinase 1 Alpha 1 (CSNK1A1), Fibronectin 1 (FN1), Growth Arrest-Specific 7 (GAS7), Glycoprotein Non-Metastatic Melanoma Protein B (GPNMB), HAUS Augmin Complex Subunit 3 (HAUS3), LDLR-Fucosylated, T Cell Recognized Melanoma Antigen 2 (MART2), Myostatin (MSTN), Melanoma-Associated Antigen (Mutated) 1 (MUM-1-2-3), Poly(A) Polymerase γ (neo-PAP), Myosin I Class, Protein Phosphatase 1 Regulatory Subunit 3B (PPP1R3B), Peroxiredoxin-5 (PRDX5), Receptor-Type Tyrosine-Protein Phosphatase Kappa (PTPRK), N-Ras (Transforming Protein N-Ras), Retinoblastoma-Associated Factor 600 (RBAF600), Sirtuin 2 (SIRT2), SNRPD1, Triosephosphate Isomerase, Oculocutaneous Albinism Type 1 Protein (OA1), Member of the RAS Oncogene Family (RAB38), Tyrosinase-Related Protein 1-2 (TRP-1-2), Melanoma Antigen gp75 (gp75), Tyrosinase, MART-1, Glycoprotein 100 Melanoma Antigen (GP100), N-Acetylglucosaminyltransferase V Gene (GnTVf), Lymphocyte Antigen 6 Complex Locus K (LY6K), MAGE-A10, MAGE-A12, MAGE-C2, NA88-A, TRAG-3, PDZ Binding Kinase (pbk), Caspase-8 (CASP-8), Sarcoma Antigen 1 (SAGE), Breakpoint Cluster Region-Abelson Oncogene (BCR-ABL), Leukemia Fusion Protein, dek-can, Elongation Factor Tu GTP Binding Domain 2 (EFTUD2), ETS Variant Gene 6/Acute Myeloid Leukemia Fusion Protein (ETV6-AML1), FLT3-ITD (FMS-Like Tyrosine Kinase 3 Internal Tandem Duplication), Cyclin A1, FNIII Domain Containing 3B (FDNC3B), Promyelocytic Leukemia/Retinoic Acid Receptor Alpha Fusion Protein (pml-RARα), MAGE-C1, Membrane Protein Selective Splicing Isoform (D393-CD20), MAGE-A4, MAGE-A3.

This list encompasses a wide array of tumor-associated antigens and epitopes, highlighting the extensive potential targets for the vaccine design to achieve efficient and specific immune responses.

The infectious diseases can be caused by bacterial infections, viral infections, and other pathogenic microorganisms such as rickettsia, chlamydia, and mycoplasma.

The viral pathogens include, but are not limited to:Dengue virus, Ebola virus, Epstein-Barr virus (EBV), Hepatitis A virus, Hepatitis B virus, Hepatitis C virus, Hepatitis D virus, Human Immunodeficiency Virus (HIV), Herpes Simplex Virus 1 (HSV1), Herpes Simplex Virus 2 (HSV2), Cytomegalovirus (CMV), Influenza A virus, Marburg virus, Respiratory Syncytial Virus (RSV), SARS Coronavirus (SARS-CoV), West Nile virus, Human Papillomavirus (HPV), Human Rhinovirus (HRV), Rabies virus (HRV), Zika virus.

The bacterial pathogens include, but are not limited to: Acinetobacter baumannii, Burkholderia cepacia, Bacteroides fragilis, Chlamydia trachomatis, Citrobacter freundii, Campylobacter jejuni, Escherichia coli, Enterobacter aerogenes, Enterobacter cloacae, type BHaemophilus influenzae, Helicobacter pylori, Klebsiella oxytoca, Klebsiella pneumoniae (MDR/CRE), Legionella pneumophila, Neisseria meningitidis, Neisseria gonorrhoeae, Pseudomonas aeruginosa, Salmonella typhi, Salmonella paratyphi, Salmonella typhimurium, Serratia marcescens, Shigella flexneri, Stenotrophomonas maltophilia, Yersinia pseudotuberculosis, Bacillus subtilis, Clostridium neoformans, Clostridium difficile, Clostridium perfringens, Corynebacterium spp., Enterococcus faecalis, Enterococcus faecium, Vancomycin-Resistant Enterococci (VRE), Listeria monocytogenes, Mycobacterium avium, Mycobacterium tuberculosis, Mycobacterium leprae, Nocardia farcinica, Propionibacterium acnes, Staphylococcus aureus, Methicillin-Sensitive Staphylococcus aureus (MSSA), Methicillin-Resistant Staphylococcus aureus (MRSA), Staphylococcus epidermidis, Streptococcus pyogenes, Group A Streptococcus, Group B Streptococcus (Streptococcus agalactiae), Group C Streptococcus.

The fungal pathogens include, but are not limited to: Aspergillus spp., Blastomyces spp., Candida albicans, Candida glabrata, Candida guilliermondii, Candida krusei, Candida parapsilosis, Candida tropicalis, Cryptococcus spp., Fusarium spp., Mucor spp., Saccharomyces spp., Pneumocystis jirovecii (carinii).

This comprehensive list highlights the wide range of potential viral, bacterial, and fungal pathogens that can cause infectious diseases, underscoring the importance of developing vaccines and therapies that target these diverse organisms.

In some embodiments, the tumor antigen or antigen epitope peptide is selected from antigens caused by gene mutations, tissue-specific differentiation antigens, overexpressed antigens, cancer-testis antigens, common antigens, and oncogenic viral antigens.

Among them, the antigen caused by the gene mutation in the tumor antigen is optional from one or more of p53, ras, β-catenin, CDK4, CDC27, and α-actinin-4.

Among them, the tissue-specific differentiation antigen in the tumor antigen is optional from one or more of Tyrosinase, TRP1/gp75, TRP2, gp100, Melan-A/MART1, gangliosides, and PSMA.

The overexpressed antigen in the tumor antigen can be selected from one or more of HER2, WT1, EphA3, EGFR, and CD20.

The cancer testis antigen in the tumor antigen is optional from one or more of MAGE, BAGE, GAGE, and NY-ESO-1.

The universal antigen in the tumor antigen can be selected from one or more of Telomerase, Survivin.

The oncogenic virus derived antigen in the tumor antigen is optional from one or more of EBV, HPV.

It should be noted that the nucleic acid molecules mentioned here include either strand of the complementary double-stranded molecule or both. For convenience, while only one strand is usually provided, the complementary strand is also disclosed. The nucleic acid sequences include DNA or RNA forms, with disclosure of one implying the other.

When connecting the nucleic acid molecule to a vector, the molecule can be directly or indirectly linked to control elements on the vector, as long as these elements can control the translation and expression of the nucleic acid molecule. These control elements may be intrinsic to the vector or exogenous. The term "operably linked" means connecting an exogenous gene to the vector so that the control elements within the vector, such as transcriptional and translational control sequences, can exert their intended regulatory functions on the exogenous gene. Common vectors include plasmids, viral vectors, bacteriophages, etc.

Upon introduction of the vector into suitable host cells, effective expression of the protein, antibody, or conjugate can be achieved under the regulation of the control system, allowing for large-scale production of these molecules in vitro.

The invention encompasses a series of nucleic acid molecules containing the open reading frame (ORF). These ORFs include the coding region for the antigen element-(linker)-E3 ubiquitin ligase recruiting ligand element structure, with or without a signal peptide sequence coding region.

The nucleic acid molecules can contain a 5' untranslated region (UTR), ORF, and 3' UTR, connected sequentially. They may also include a promoter. A DNA template connected sequentially by a promoter, 5' UTR, ORF, and 3' UTR can be transcribed to obtain mRNA. Transcription can be carried out using existing in vitro transcription methods and related kits. Additionally, the nucleic acid molecules can include a polyA tail.

The detailed descriptions and specific sequences provided ensure comprehensive understanding and implementation of the invention for enhanced antigen presentation and vaccine efficacy.

In one embodiment, the promoter is T7 or SP6.

In one embodiment, the nucleotide sequence of the 5' untranslated region is as follows (Seq ID No.18):

AGGCAAAAATCAAAATCAATCATCATCACAACATCAACAATCAATCATCAACACAT
CATCAAGACAGCCACC

In one embodiment, the nucleotide sequence of the 3' untranslated region is as follows (Seq ID No.19):

TGATGAGCTCGCTTTCTTGCTGTCCAATTTCTATTAAAGGTTCCTTTGTTCCCTAAGT
CCAACTACTAAACTGGGGGATATTATGAAGGGCCTTGAGCATCTGGATTCTGCCTAATAA
AAAACATTTATTTTCATTGCTGCGTCGAGAGCTCGCTTTCTTGCTGTCCAATTTCTATTAA
AGGTTCCTTTGTTCCCTAAGTCCAACTACTAAACTGGGGGATATTATGAAGGGCCTTGAG
CATCTGGATTCTGCCTAATAAAAAACATTTATTTTCATTGC

In one embodiment, the nucleotide sequence of the polyA is as follows (Seq ID No.20):

AAAAAAAAAAAAAAAAAAAAAAAAAAAAAAAAAAAAAAAAAAAAAAAAAAGT
CTTCAAAAAAAAAAAAAAAAAAAAAAAAAAAAAAAAAAAAAAAAAAAAAAAAAA

The aforementioned nucleic acids, proteins, or polypeptides could serve as active ingredients for drugs or vaccines for preventing and/or treating diseases. Generally, a skilled person in the field could use the aforementioned proteins as antigenic active ingredients to prepare protein or polypeptide vaccines. These vaccines used the aforementioned proteins as antigenic components, along with pharmaceutically acceptable excipients or auxiliary components.

In vaccine preparation, adjuvants are often added to enhance the immune response to the vaccine. The adjuvants includes incomplete Freund's adjuvant, complete Freund's adjuvant, aluminum hydroxide adjuvant, aluminum phosphate adjuvant, emulsion adjuvant, liposome adjuvant, and microbial adjuvant.

Naturally, based on the proteins described in this invention, it is easy to obtain antibodies against these proteins. These antibodies could be polyclonal or monoclonal; monoclonal antibodies are preferred. These antibodies could also form conjugates with conjugate parts. The conjugate parts are selected from radionuclides, drugs, toxins, cytokines, enzymes, fluoresceins, carrier proteins, or biotin. Antibodies that specifically binds to the aforementioned proteins could be used for drug preparation and for immunoassays related to the aforementioned proteins.

Additionally, this invention also includes the genes encoding the aforementioned proteins. These genes could be used for expressing the aforementioned proteins or antibodies. They could also be operably loaded into expression vectors to prepare vector vaccines or vector drugs. The expression could be performed in commonly used vectors such as plasmid vectors, adenoviral vectors, lentiviral vectors, or adeno-associated viral vectors. When using adenoviral vectors, replication-defective adenoviral vectors were generally used.

For example, when the nucleic acid molecules were DNA, the transcribed mRNA could be formulated into mRNA vaccines with pharmaceutically acceptable excipients or auxiliary components. The auxiliary components could be nanocarriers for the mRNA. The commonly used nanocarriers are lipid nanocarriers. For example, the lipid nanocarriers are made from at least one of the following materials: DOTAP, DOTMA, DOTIM, DDA, DC-Chol, CCS, diC14-amidine, DOTPA, DOSPA, DTAB, TTAB, CTAB, DORI, DORIE and its derivatives, DPRIE, DSRIE, DMRIE, DOGS, DOSC, LPLL, DODMA, DDAB, Dlin-MC3-DMA, CKK-E12, C12-200, DSPC, DMG-PEG, DOPE, phosphatidylethanolamine (PE), phosphatidylcholine (PC), and cholesterol (Chol).

Preferably, the lipid materials used for preparing the nanocarriers are amphiphilic lipid materials or cationic lipid materials. These lipid materials carries a positive charge under acidic conditions, which allows them to encapsulate mRNA molecules through electrostatic adsorption with the phosphate groups of nucleic acids, forming mRNA-lipid complexes. These mRNA-lipid complexes could be adsorbed by the negatively charged cell membrane and then transferred into cells through membrane fusion or endocytosis, where they could be further expressed to exert the immune effect of the vaccine.

Generally, the preparation of the aforementioned mRNA vaccine could use microfluidic devices to allow the mRNA and lipid materials to self-assemble into mRNA-lipid complexes. Alternatively, pre-prepared nanocarriers could be incubated with mRNA to form mRNA-lipid complexes. The following examples provide a detailed description of the invention.

### Example 1: Construction of mRNA Transcription Vector Template Containing E3 Ubiquitin Ligase Ligand Antigen Elements

In this example, the DNA template for mRNA transcription consisted of a promoter, a 5' untranslated region, a signal peptide sequence, an antigen coding sequence, a linker sequence, a recruiting ligand sequence for the E3 ubiquitin ligase, a 3' untranslated region, and a poly(A) tail. These elements were sequentially connected and inserted into the corresponding positions in a plasmid. The sequence was verified by sequencing, resulting in the mRNA transcription template DNA. The signal peptide sequence, antigen coding sequence, linker sequence, and E3 ubiquitin ligase recruiting ligand sequence could be provided in two separate sequences or in a single sequence.

The promoter was T7 or SP6.

Using ovalbumin (OVA) as a model antigen, recruiting ligands P53B, PMI, VHLL, and keap1B from three E3 ubiquitin ligases-PMI, VHL, and Keap1-were chosen to construct and prepare mRNAs encoding full-length OVA protein. These included OVA-mRNA (control), OVA-P53B mRNA, OVA-PMI mRNA, OVA-VHLL mRNA, and OVA-keap1B mRNA. A positive control OVA-H2Db mRNA was also constructed. The antigen coding regions were inserted into the corresponding positions in the plasmid vector and verified by sequencing. The results are shown in Table 2.

**Table 2: Sequence Information of mRNA Containing E3 Ubiquitin Ligase Ligand Antigen Elements**

| **mRNA ID** | **mino Acid Sequence Corresponding to E3 Ubiquitin Ligase Ligand Coding Sequence** |
|---|---|
| OVA mRNA | / |
| OVA-H2Db mRNA | / |
| OVA-P53B mRNA | ETFSDLWKLL |
| OVA-PMI mRNA | TSFAEYWNLLSP |
| OVA-VHLL mRNA | ALAPYIP |
| OVA-keap1B mRNA | LDPETGEYL |

### Example 2: In Vitro Transcription of mRNA Containing E3 Ubiquitin Ligase Ligand Antigen Elements

The mRNA transcription template DNA from Example 1 was inserted into the pUC57 vector to prepare plasmid DNA. Following the instructions from "Molecular Cloning: A Laboratory Manual (4th Edition)" and commercial kits for restriction enzymes and DNA purification, the plasmid DNA was digested to linearize the plasmid DNA template and then purified. The concentration and purity of the plasmid DNA and linearized DNA template were measured using spectrophotometry and gel electrophoresis, which also confirmed complete linearization.

mRNA synthesis was carried out using RNA polymerase, NTPs, and cap analogs. The in vitro transcription of precursor mRNA was performed according to the kit instructions (Vazyme TR101-02). The specific procedures are as follows:
According to Table 3, the relevant reactants were added to a 1.5 mL centrifuge tube in the specified amounts and order. The mixture was pipetted up and down three times or gently tapped at the bottom of the tube to mix. The reaction mixture was briefly centrifuged to collect it at the bottom of the tube. The tube was then incubated in a 37°C water bath for 4 hours. After incubation, 1 µL of DNase was added and incubated at 37°C for 15 minutes. Then, 179 µL of nuclease-free water was added, followed by 200 µL of phenol/chloroform/isoamyl alcohol (25:24:1) solution for mRNA extraction. The mixture was centrifuged at 15,000 rpm for 10 minutes, and the aqueous phase was collected. To precipitate the mRNA, 200 µL of 5 M ammonium acetate solution was added, and the mixture was stored at 4°C overnight. The next day, the mixture was centrifuged at 4°C and 15,000 rpm for 10 minutes, and the supernatant was carefully removed. The precipitate was washed with 1 mL of 70% ethanol, centrifuged again at 4°C and 15,000 rpm for 10 minutes, and the supernatant was carefully removed. The precipitate was air-dried and then resuspended in 20 µL of nuclease-free water to obtain the mRNA solution.

The purity of the mRNA was checked using agarose gel electrophoresis or a nucleic acid fragment analyzer.

**Table 3: mRNA Transcription Reaction Mixture Composition**

| **Component** | **2 mL Prescription** |
|---|---|
| ATP (100 mM) | 150 µL |
| GTP (100 mM) | 150 µL |
| CTP (100 mM) | 150 µL |
| UTP (100 mM) | 150 µL |
| Clean Cap^{®} AG | 20 µL |
| Linear DNATemplet | 0.5 mg |
| 10×Translation Buffer | 200 µL |
| Murine RNase Inhibitor (40 U/µL) | 100 µL |
| T7 RNA Polymerase (50 U/µL) | 200 µL |
| Pyrophosphatase, Inorganic (yeast) (0.1 U/µL) | 10 µL |
| RNase-free water | Add to 2 mL |

### Example 3: Preparation of mRNA Lipid Nanoparticles (mRNA-LNPs)

As shown in Table 4, mRNA lipid nanoparticles can be prepared according to the prescription requirements for mRNA and target organs. The specific method is as follows:
Dissolve the lipid materials in ethanol solution and then mix with the corresponding mRNA solution to allow self-assembly into mRNA-lipid nanoparticles. The ethanol is then removed using a tangential flow system, followed by sterilization filtration to obtain the final product.
(1) Preparing solution: Dissolve cationic lipids (MC3, DTAB, DC-Chol, CTAB, DOTMA, DDA, DOTAP), helper lipids (DSPC or DOPE), cholesterol (Chol), and DMG-PEG2000 in anhydrous ethanol to a concentration of 10 mg/mL for ionizable lipids to obtain the lipid solution. The molar ratio of cationic lipid, helper lipid, cholesterol, and DMG-PEG2000 is 50:10:38.5:1.5. Dilute the mRNA in PBS buffer (RNase-free water) to a suitable concentration for use.
(2) Preparing LNP: Mix the lipid solution obtained in step (1) with different concentrations of mRNA solutions to achieve the samples with various mass ratios of cationic lipid to mRNA as shown in Table 4. This mixing is performed in a microfluidic device (Mienna Instruments, Shanghai). The mixing parameters are: the volume ratio of lipid solution to mRNA solution is 1:3, and the total flow rate is 9 mL/min.
(3) Ultrafiltration: Dilute the primary LNP preparation made by microfluidic mixing 25 times with PBS buffer and ultrafilter to the original volume to obtain the final LNP preparation. Ethanol is removed during ultrafiltration. The ultrafiltration parameters are: 100 kDa filter membrane, 0.2 MPa pressure, and 100-200 rpm speed.

Using the above method, mRNA lipid nanoparticles containing OVA-mRNA, OVA-H2Db mRNA, OVA-P53B mRNA, OVA-PMI mRNA, OVA-VHLL mRNA, and OVA-keap1B mRNA were prepared according to the prescription and mass ratio in Table 4, along with blank lipid nanoparticles without mRNA. The particle size, zeta potential, and mRNA encapsulation efficiency of the seven types of mRNA lipid nanoparticles were measured using specific detection methods.

We measured a specific volume of mRNA LNP colloidal solution and diluted it with purified water to an mRNA concentration of 0.01 mg/ml. The particle size and zeta potential of the mRNA LNPs were measured using a laser particle size analyzer (n=3, three measurements for each formulation).

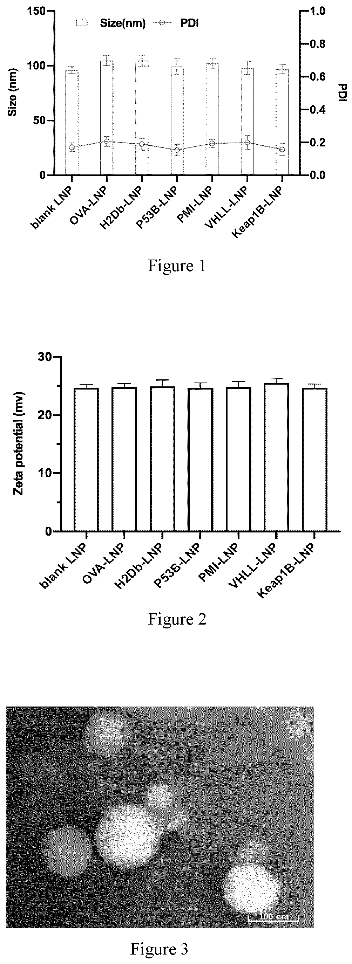

The results showed that the particle sizes of the seven mRNA LNP formulations were approximately 100 nm (Figure 1). The PDI values were around 0.2, and the zeta potentials were about 25 mV (Figure 2), indicating good uniformity.

We used the Quant-iT^{™} RiboGreen^{™} assay kit to determine the encapsulation efficiency, with results shown in Table 5. The results indicated that the mRNA-LNPs had good nanoparticle properties, with encapsulation efficiencies exceeding 95%, providing good protection for the mRNA.

We observed the morphology of the prepared OVA-P53B mRNA LNPs under TEM and took photographs (Figure 3). The TEM results showed that the OVA-P53B mRNA LNPs had a quasi-spherical structure with a smooth appearance. The particle size was consistent with the results from the particle size analyzer. Other mRNA LNPs, such as OVA-PMI, OVA-VHLL, and OVA-keap1B, showed similar morphological results under TEM.

**Table 4 Formulations of mRNA Lipid Nanoparticles**

| Prescription | LNP solution | 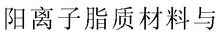 mRNA 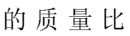 Mass ratio of ionizable lipid material and mRNA |
|---|---|---|
| 1 | MC3, DSPC, DMG-PEG, Cholesterol | 15:1 |
| 2 | DTAB, DSPC, DMG-PEG, Cholesterol | 10:1 |
| 3 | DC-Chol, DOPE, DMG-PEG, Cholesterol | 25:1 |
| 4 | CTAB, DOPE, DMG-PEG, Cholesterol | 30:1 |
| 5 | DOTMA, DSPC, DMG-PEG, Cholesterol | 50:1 |
| 6 | DDA, DOPE, DMG-PEG, Cholesterol | 5:1 |
| 7 | DOTAP, DOPE, DMG-PEG, Cholesterol | 15:1 |

**Table 5 Encapsulation Efficiency Results**

| mRNA-LNP | Encapsulation Efficiency (%) |
|---|---|
| OVA mRNA-LNP | 95.87 |
| OVA-H2Db mRNA-LNP | 98.02 |
| OVA-P53B mRNA-LNP | 97.65 |
| OVA-PMI mRNA-LNP | 96.61 |
| OVA-VHLL mRNA-LNP | 95.11 |
| OVA-keap1B mRNA-LNP | 97.27 |

The seven prepared mRNA lipid nanoparticle formulations exhibited good size uniformity and stability. The zeta potentials were moderate and uniform.

### Example 4: In Vivo T Cell Activation by mRNA Lipid Nanoparticles Containing E3 Ubiquitin Ligase Ligand Antigen Elements

We administered lipid nanoparticles encapsulating OVA-mRNA and OVA-P53B mRNA, prepared as described in Example 3, to mice. The mRNA concentration in the lipid nanoparticle samples was 0.1 mg/mL. The procedure was as follows:
After one week of adaptive feeding, C57BL/6 mice were divided into three groups: control, OVA, and OVA-P53, with four mice in each group. Mice were immunized via intravenous injection. The control group received 100 µL of saline, while the OVA and OVA-P53 groups received 100 µL of the respective mRNA lipid nanoparticle samples. After 36 hours, all mice were euthanized and dissected to collect spleen and lymph node tissues. The tissues were processed and filtered to obtain single-cell suspensions. The suspensions were centrifuged at 400 g, treated with red blood cell lysis buffer at room temperature, and centrifuged again. The supernatant was discarded. T cells were stained with antibodies (anti-CD45, anti-CD3ε, anti-CD4, anti-CD8, anti-CD25, and anti-CD69) at room temperature, centrifuged, and the supernatant was discarded. The cells were resuspended and analyzed using flow cytometry.

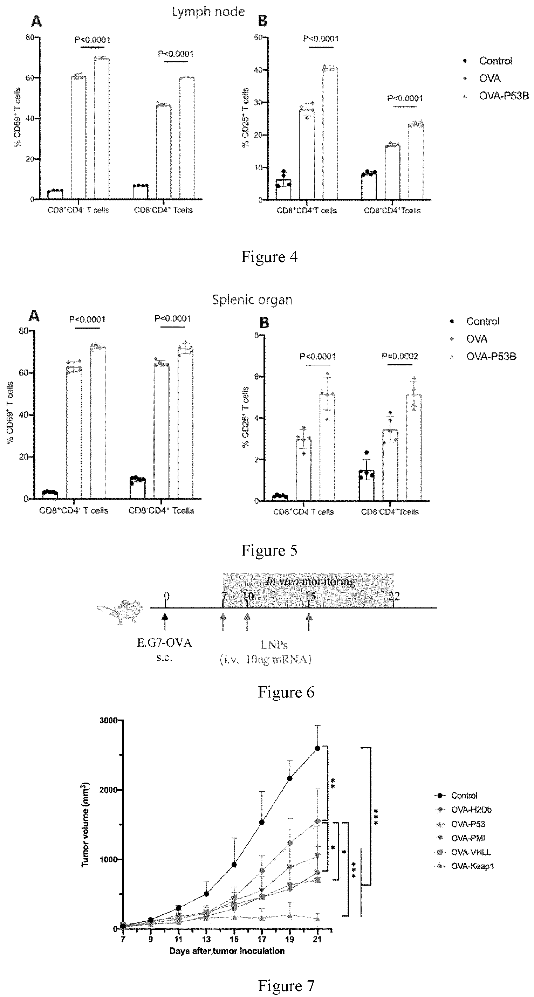

The results are shown in Figures 4 and 5:
Figure 4 displays the immune cell detection results in lymph nodes. Figure 4A shows the data for early activation of CD8+ and CD4+ T cells, and Figure 4B shows the data for late activation of CD8+ and CD4+ T cells. The data indicate that the OVA-P53B mRNA lipid nanoparticles, which incorporate the E3 ubiquitin ligase recruitment ligand P53B, activated CD8+ and CD4+ T cells in lymph nodes more effectively than the OVA-mRNA lipid nanoparticles without the E3 ubiquitin ligase recruitment ligand P53B.

Figure 5 presents the immune cell detection results in the spleen. Figure 5A shows the data for early activation of CD8+ and CD4+ T cells, and Figure 5B shows the data for late activation of CD8+ and CD4+ T cells. The data indicate that the OVA-P53B mRNAlipid nanoparticles, which incorporate the E3 ubiquitin ligase recruitment ligand P53B, activated CD8+ and CD4+ T cells in the spleen more effectively than the OVA-mRNA lipid nanoparticles without the E3 ubiquitin ligase recruitment ligand P53B.

These findings suggest that the mRNA lipid nanoparticles with the E3 ubiquitin ligase recruitment ligand P53B effectively mediated T cell activation in mice, showing significantly better results than the mRNA lipid nanoparticles without the E3 ubiquitin ligase ligand antigen elements.

### Example 5: In Vivo Anti-Tumor Effects of mRNA Lipid Nanoparticles Containing E3 Ubiquitin Ligase Ligand Antigen Elements Against E.G7-OVA Tumors

We administered lipid nanoparticles encapsulating OVA mRNA, OVA-H2Db mRNA, OVA-P53B mRNA, OVA-PMI mRNA, OVA-VHLL mRNA, and OVA-keap1B mRNA, prepared as described in Example 3, to mice. The mRNA concentration in the lipid nanoparticle samples was 0.1 mg/mL.
1. To investigate the effect of the mRNA lipid nanoparticles on tumor volume, we selected 5-6 week-old male C57BL/6 mice and randomly divided them into groups of six. We examined the anti-tumor effects of the OVA mRNA lipid nanoparticles containing E3 ubiquitin ligase ligand antigen elements on E.G7-OVA tumor-bearing mice.

After one week of adaptive feeding, we adjusted the density of E.G.7-OVA tumor cell suspension to 7×10^6 cells/mL and injected 100 µL subcutaneously into the upper right axilla of C57BL/6 mice. Seven days after inoculation, we immunized the mice intravenously according to the experimental design (Figure 6). The mRNA-LNP dosage was 10 µg mRNA per mouse (the control group received the same volume of saline). We measured tumor diameter with electronic calipers on the first day of dosing and recorded it every other day. We calculated tumor volume according to the experimental design and plotted the tumor growth volume-time curve of the tumor-bearing mice, as shown in Figure 7.

The results showed that, compared to the control group and the positive control OVA-H2Db group, the OVA mRNA vaccines containing E3 ubiquitin ligase ligand antigen elements significantly inhibited tumor growth. The OVA-P53B formulation exhibited the best tumor inhibition effect.

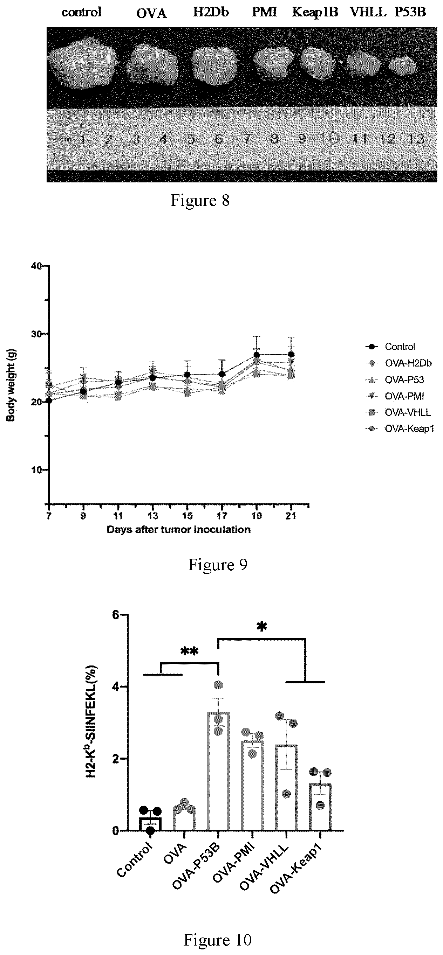

2. After the immunization experiment, we euthanized all mice and collected tumor tissues for photography and weighing to calculate the tumor inhibition rate. The results are shown in Figure 8.

The results showed that OVA-H2Db group had a tumor inhibition rate of 38.8%, indicating a certain degree of tumor inhibition. The tumor inhibition rates of OVA-PMI, OVA-Keap1B, and OVA-VHLL were 51.1%, 66.8%, and 70.5%, respectively, showing good anti-tumor effects. The OVA-P53B group had a tumor inhibition rate of 84.8%, indicating a superior anti-tumor effect.

3. To preliminarily evaluate the safety of the administration, we weighed the tumor-bearing mice with an electronic balance on the first day of dosing and recorded the weight every other day. We plotted the body weight-time curve of the tumor-bearing mice, as shown in Figure 9.

The results showed an increasing trend in body weight across all groups during the treatment period. No significant weight loss was observed, indicating no apparent toxic side effects and good in vivo safety of the formulations.

4. To assess the antigen presentation and specific CTL generation mediated by the mRNA lipid nanoparticles in mice, we euthanized all mice after the immunization experiment and collected lymph nodes, spleen, and tumor tissues. We processed the tissues, filtered to obtain single-cell suspensions, centrifuged, and treated with red blood cell lysis buffer at room temperature, followed by a second centrifugation and discarding of the supernatant. We stained the cells with antibodies for various immune cells such as DCs, macrophages, and effector T cells (including anti-CD45, anti-CD11c, anti-CD3ε, anti-CD4, anti-CD8, anti-H-2Kb bound to SIINFEKL, and H2Kb OVA tetramer), centrifuged, and discarded the supernatant. We resuspended the cells for flow cytometry analysis.

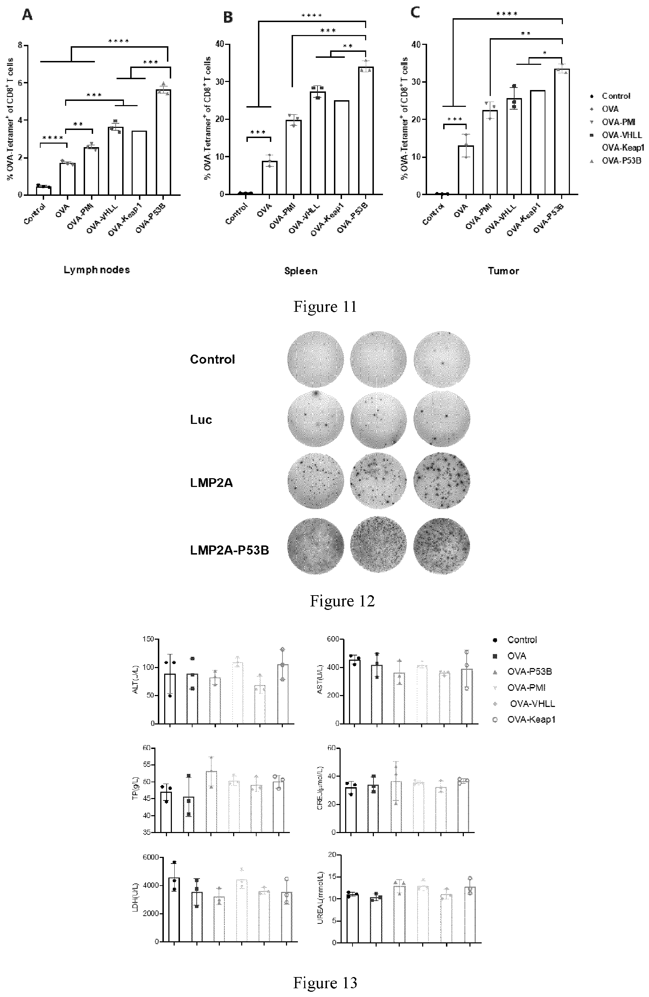

Figures 10 and 11 present the results of antigen presentation and specific CTL generation mediated by the mRNA lipid nanoparticles in mice. Figure 11A shows the specific CTL detection results in lymph nodes, Figure 11B in the spleen, and Figure 11C in tumor tissues. The results indicate that, compared to OVA mRNA without E3 ubiquitin ligase ligand antigen elements, the mRNA lipid nanoparticles containing OVA-P53B mRNA, OVA-PMI mRNA, OVA-VHLL mRNA, and OVA-keap1B mRNA mediated stronger antigen presentation and generated more specific CTLs in mice. Among these, the OVA-P53B formulation exhibited the best effect.

### Example 6: In Vivo Anti-EBV Tumor Effects of P53 on EBV LMP2A @mRNA Lipid Nanoparticles

The amino acid sequence of the EBV virus LMP2A antigen is as follows (Seq ID No.21):

MGSLEMVPMGAGPPSPGGDPDGYDGGNNSQYPSASGSSGNTPTPPNDEERESNEEPPP
PYEDPYWGNGDRHSDYQPLGTQDQSLYLGLQHDGNDGLPPPPYSPRDDSSQHIYEEAGRG
SMNPVCLPVIVAPYLFWLAAIAASCFTASVSTVVTATGLALSLLLLAAVASSYAAAQRKLLT
PVTVLTAVVTFFAICLTWRIEDPPFNSLLFALLAAAGGLQGIYVLVMLVLLILAYRRRWRRLT
VCGGIMFLACVLVLIVDAVLQLSPLLGAVTVVSMTLLLLAFVLWLSSPGGLGTLGAALLTL
AAALALLASLILGTLNLTTMFLLMLLWTLVVLLICSSCSSCPLSKILLARLFLYALALLLLAS
ALIAGGSILQTNFKSLSSTEFIPNLFCMLLLIVAGILFILAILTEWGSGNRTYGPVFMCLGGLLT
MVAGAVWLTVMSNTLLSAWILTAGFLIFLIGFALFGVIRCCRYCCYYCLTLESEERPPTPYRN
TV

We prepared mRNA containing the above LMP2A antigen sequence using the methods described in Examples 1 and 2. We then prepared lipid nanoparticles encapsulating LMP2A-mRNA, LMP2A-P53B mRNA, and LUC mRNA using the method described in Example 3, with a cationic lipid to mRNA mass ratio of 15:1. The mRNA concentration in the lipid nanoparticle samples was 0.1 mg/mL. We investigated the in vivo anti-tumor effects of the LMP2A-P53B mRNA lipid nanoparticles containing the E3 ubiquitin ligase ligand antigen element P53B using the method disclosed in Example 5.

We examined the anti-tumor effect of the LMP2A-P53B mRNA lipid nanoparticles containing the E3 ubiquitin ligase ligand antigen element on the production of specific T cells using the ELISpot assay.

After completing the immunization experiment, we euthanized and dissected all mice, collecting their spleens. We processed and filtered the spleens to obtain single-cell suspensions, centrifuged them, and treated them with red blood cell lysis buffer at room temperature. After a second centrifugation, we discarded the supernatant to obtain spleen lymphocyte suspensions, which were then analyzed.

As shown in Figure 12, the mRNA lipid nanoparticles containing the LMP2A antigen mediated the production of specific CTLs in mice. The LMP2A-P53B mRNA lipid nanoparticles with the E3 ubiquitin ligase ligand antigen element showed more significant effects.

These results indicate that, compared to the control group, the model mRNA group (LUC mRNA), and the group containing only the LMP2A antigen (LMP2A mRNA), the LMP2A-P53B mRNA lipid nanoparticles containing the E3 ubiquitin ligase ligand antigen element P53B induced more CTL production and had a stronger inhibitory effect on tumor growth, resulting in a higher tumor inhibition rate.

### Example 7: Evaluation of the Safety of mRNA Lipid Nanoparticles Containing E3 Ubiquitin Ligase Ligand

In this example, we assessed the safety of the mRNA lipid nanoparticles prepared in Example 3 in mice.

After one week of adaptive feeding, C57BL/6 mice were intravenously immunized according to the experimental design (Figure 6 - Immunization Protocol Diagram). The dosage was 10 µg of mRNA per mouse. Seven days post-administration, blood samples were collected from the mice, and mouse plasma was prepared. Serum levels of ALT, AST, TP, CRE, LDH, and UREA were measured using a biochemical analyzer.

As shown in Figure 13, there were no significant changes in the levels of ALT, AST, TP, CRE, LDH, and UREA in the serum of mice immunized with the various mRNA lipid nanoparticles compared to the control group.

This indicates that there were no significant changes in liver and kidney function indicators after administration of the mRNA lipid nanoparticles containing the E3 ubiquitin ligase ligand antigen element. Thus, these mRNA lipid nanoparticles demonstrated good safety.
It should be noted that the specific features, structures, materials, or characteristics described in this specification may be combined in suitable ways in one or more embodiments. Furthermore, different embodiments and features described in this specification can be combined and integrated by those skilled in the art without contradiction.

## Claims

1. A nucleic acid molecule, comprising:
at least one open reading frame;
wherein the open reading frame contains at least one antigen element and at least one E3 ligand element; the E3 ligand element is a binding or recruiting ligand of E3 ubiquitin ligase; and the binding or recruiting ligand of E3 ubiquitin ligase binds to one or more E3 ubiquitin ligases.

2. The nucleic acid molecule of claim 1, wherein the E3 ubiquitin ligase is selected from RING-type, HECT-type, and RBR-type.

3. The nucleic acid molecule of claim 1, wherein the E3 ubiquitin ligase is optionally selected from VHL, MDM2, CRBN, IAPs, RNF, β-TrCP, DCAF, Keap1, or their truncated or extended forms; furthermore, the E3 ligand binds to one or more E3 ubiquitin ligases.

4. The nucleic acid molecule of claim 3, wherein the binding or recruiting ligand of Keap1 corresponds to the amino acid sequence LDPETGEYL or a sequence with more than 60% homology.

5. The nucleic acid molecule of claim 3, wherein the binding or recruiting ligand of β-TrCP corresponds to the amino acid sequence DRHDSGLDSM or a sequence with more than 60% homology.

6. The nucleic acid molecule of claim 3, wherein the binding or recruiting ligand of VHL corresponds to the amino acid sequence LAP(OH)YI or ALAPYIP or a sequence with more than 60% homology.

7. The nucleic acid molecule of claim 3, wherein the binding or recruiting ligand of MDM2 corresponds to at least one amino acid sequence selected from ETFSDLWKLL, TSFAEYWNLLSP, LTFEHYWAQLTS, TNWYANLEKLLR, TAWYANFEKLLR, DWWPLAFEALLR, CNCKAPETALCARRCQQH, or CNCKAPETFLCYWRCLQH, or a sequence with more than 60% homology.

8. The nucleic acid molecule of any of claims 1 to 7, wherein the antigen element and E3 ligand element are connected in any form selected from E3 ligand-antigen, antigen-E3 ligand, E3 ligand-antigen-E3 ligand, or antigen-E3 ligand-antigen; the antigen element and E3 ligand element are connected via a linker.

9. The nucleic acid molecule of claim 8, wherein the linker has an amino acid sequence selected from GGGGS, (GGGGS)3, (GGGGS)6, (GGS)10, or (GSG)10.

10. The nucleic acid molecule of any of claims 1 to 9, wherein the antigen element includes at least one antigen epitope.

11. The nucleic acid molecule of claim 10, wherein the antigen element is a natural antigen protein, antigen polypeptide, or a truncated form thereof.

12. The nucleic acid molecule of claim 10, wherein the antigen element includes 1 to 20 antigen epitopes.

13. The nucleic acid molecule of claim 10, wherein the antigen epitope is a T cell antigenic determinant or T cell antigen epitope.

14. The nucleic acid molecule of claim 10, wherein the antigen epitope is derived from tumor antigen peptides, autoimmune disease antigen peptides, or pathogenic microorganism antigen peptides.

15. The nucleic acid molecule of claim 14, wherein the tumor antigens or antigen peptides are selected from antigens caused by gene mutations, tissue-specific differentiation antigens, overexpressed antigens, cancer-testis antigens, universal antigens, or oncogenic viral antigens; the antigens caused by gene mutations are selected from p53, ras, β-catenin, CDK4, CDC27, or α actinin-4; the tissue-specific differentiation antigens are selected from Tyrosinase, TRP1/gp75, TRP2, gp100, Melan-A/MART1, gangliosides, or PSMA; the overexpressed antigens are selected from HER2, WT1, EphA3, EGFR, or CD20; the cancer-testis antigens are selected from MAGE, BAGE, GAGE, or NY-ESO-1; the universal antigens are selected from Telomerase or Survivin; and the oncogenic viral antigens are selected from EBV, HPV, HBV, HCV, human herpesvirus, or Merkel cell polyomavirus.

16. The nucleic acid molecule of any of claims 1 to 15, wherein the nucleic acid molecule further comprises a signal peptide coding region.

17. The nucleic acid molecule of any of claims 1 to 16, wherein the nucleic acid molecule further comprises a promoter, 5' untranslated region, 3' untranslated region, and polyA; and the promoter is either a T7 or SP6 promoter.

18. The nucleic acid molecule of any of claims 1 to 17, wherein the nucleic acid is selected at least one from DNA, ASO, siRNA, miRNA, mRNA, or aptamers.

19. A nucleic acid vaccine, comprising:
any of the nucleic acid molecules of claims 1 to 18; and
optionally pharmaceutically acceptable excipients or auxiliary ingredients;
wherein the nucleic acid vaccine is an mRNA vaccine; the auxiliary ingredients are nanoparticle carriers for mRNA; and the excipients are selected from at least one of injectable buffer media, lyophilization protectants, or cryoprotectants.

20. The nucleic acid vaccine of claim 19, wherein the nanoparticle carriers are selected from liposomes, nanoparticles, microspheres, or lipid nanoparticles.

21. The nucleic acid vaccine of claim 19, wherein the nanoparticle carriers are prepared from at least one lipid material selected from DOTAP, DOTMA, DOTIM, DDA, DC-Chol, CCS, diC14-amine, DOTPA, DOSPA, DTAB, TTAB, CTAB, DORI, DORIE and its derivatives, DPRIE, DSRIE, DMRIE, DOGS, DOSC, LPLL, DODMA, DDAB, Dlin-MC3-DMA, CKK-E12, C12-200, DSPC, DMG-PEG, DOPE, phosphatidylethanolamine, phosphatidylcholine, or cholesterol.

22. The nucleic acid vaccine of claim 19, wherein the mRNA vaccine is formed by self-assembling mRNA and lipid materials using microfluidic devices, or by incubating the nanoparticle carriers with mRNA.

23. A protein, wherein the protein is encoded by any of the nucleic acid molecules of claims 1 to 18.

24. A protein or polypeptide vaccine, comprising:
the protein of claim 23 as the antigen component.

25. The protein or polypeptide vaccine of claim 24, further comprising:
pharmaceutically acceptable excipients or auxiliary ingredients; and
an adjuvant.

26. The protein or polypeptide vaccine of claim 25, wherein the adjuvant is selected from one or more of incomplete Freund's adjuvant, complete Freund's adjuvant, aluminum hydroxide adjuvant, aluminum phosphate adjuvant, emulsion adjuvant, liposome adjuvant, and microbial adjuvant.

27. A carrier, wherein the carrier carries any of the nucleic acid molecules of claims 1 to 18; and the carrier is a eukaryotic or prokaryotic carrier.

28. The carrier of claim 27, wherein the carrier is selected from one or more of plasmid vectors, adenovirus vectors, lentivirus vectors, and adeno-associated virus vectors.

29. A carrier vaccine, comprising:
active components;
wherein the active components are obtained by loading any of the nucleic acid molecules of claims 1 to 18 into the carrier of claims 27 to 28.

30. A pharmaceutical composition, comprising: any of the nucleic acid molecules of claims 1 to 18, any of the nucleic acid vaccines of claims 19 to 22, the protein of claim 23, any of the protein or polypeptide vaccines of claims 24 to 26, or any of the carrier vaccines of claim 29. It also included pharmaceutically acceptable excipients.

31. The nucleic acid molecules of claims 1 to 18, the nucleic acid vaccines of claims 19 to 22, the protein of claim 23, any of the protein or polypeptide vaccines of claims 24 to 26, any of the carrier vaccines of claim 29, and the pharmaceutical composition of claim 30 were used in the preparation of medicines for preventing or treating related diseases.
